# EUROPÄISCHE PATENTANMELDUNG

(11) **EP 2 732 779 A1**
(43) Veröffentlichungstag der Anmeldung: **21.05.2014**
(21) Anmeldenummer: 13193121.4
(22) Anmeldetag: 15.11.2013
(51) Int. Cl.: A61B 17/29, A61B 17/295

(54) **Bipolares Koagulationsinstrument**

(30) Priorität: 15.11.2012 DE 202012010938 U
(71) Anmelder: Hensler Medizintechnik, 78579 Neuhausen ob Eck (DE)
(72) Erfinder: Hensler, Ewald, 88637 Buchheim (DE)
(74) Vertreter: Patentanwälte Westphal, Mussgnug & Partner

(57) **Zusammenfassung**

Bipolares Koagulationsinstrument (10) mit einem Schaftrohr (20), mit einem am distalen Ende (20a) des Schaftrohrs (20) angeordneten Arbeitselement 830), welches zwei gegeneinander mittels zweier am proximalen Ende (20b) des Schaftrohrs (20) angeordneten, gegeneinander verschwenkbaren Griffteile (41, 42) verschwenkbare Maulteile (31, 32), welche jeweils eine Koagulationsplatte (33, 34) aufweisen, und ein mittels eines am proximalen Ende (20b) des Schaftrohrs (20)) angeordneten Abzugs (70) bewegbares Messer (50) aufweist, wobei das Messer (50) auswechselbar ist.

## Beschreibung

Die Erfindung betrifft ein bipolares Koagulationsinstrument gemäß dem Oberbegriff des Patentanspruchs 1.

Bekannt sind bipolare Koagulationsinstrumente mit einem Schaftrohr, an dessen distalem Ende ein Arbeitselement angeordnet ist, welches zwei gegeneinander verschwenkbare Maulteile mit jeweils einer Koagulationsplatte sowie ein bewegbares Messer aufweist. Die Maulteile sind mittels zweier am proximalen Ende des Schaftrohrs angeordneten, gegeneinander verschwenkbaren Griffteile gegeneinander verschwenkbar. Zusätzlich ist am proximalen Ende des Schaftrohrs ein Abzug angeordnet, mittels welchem das Messer bewegbar ist. Ein derartiges Instrument ermöglicht ein Koagulieren sowie ein anschließendes Schneiden.

Die Aufgabe der Erfindung besteht darin, ein bekanntes bipolares Koagulationsinstrument weiterzubilden, insbesondere derart, dass es eine längere Lebensdauer aufweist oder sicherer zu handhaben ist.

Die Aufgabe der Erfindung wird gelöst durch ein bipolares Koagulationsinstrument mit den Merkmalen des Patentanspruchs 1.

Vorteilhafte Ausgestaltungen und Weiterbildungen der Erfindung sind in den abhängigen Ansprüchen angegeben.

Das erfindungsgemäße bipolare Koagulationsinstrument mit einem Schaftrohr, mit einem am distalen Ende des Schaftrohrs angeordneten Arbeitselement, welches zwei gegeneinander mittels zweier am proximalen Ende des Schaftrohrs angeordneter, gegeneinander verschwenkbarer Griffteile verschwenkbare Maulteile, welche jeweils eine Koagulationsplatte aufweisen, und ein mittels eines am proximalen Ende des Schaftrohrs angeordneten Abzugs bewegbares Messer aufweist, zeichnet sich dadurch aus, dass das Messer auswechselbar ist. Gerade das Messer verschleißt bei Benutzung des bipolaren Koagulationsinstruments schnell und ist schwer zu reinigen. Ein auswechselbares Messer, wobei lediglich das im Arbeitselement angeordnete Messer ohne die im Schaftrohr angeordnete Betätigungsmechanik ausgewechselt wird, weist den Vorteil auf, dass ein Verschleißteil kostengünstig ausgetauscht werden kann, was die Lebensdauer und Benutzungsmöglichkeit des bipolaren Koagulationsinstruments verbessert.

Eine besonders bevorzugte Ausführungsform der Erfindung sieht vor, dass das Messer vom distalen Ende her auswechselbar ist. Dies vereinfacht die Handhabe des Instruments, da im Gegensatz zu bekannten Koagulationsinstrumenten, bei welchen das Messer einschließlich der Betätigungsmechanik vom proximalen Ende des Schaftrohres her aus dem Schaftrohr herausgezogen und vollständig ausgetauscht werden muss, ein schnelleres und einfacheres sowie kostengünstigeres Austauschen möglich ist.

Eine vorteilhafte Ausführungsform der Erfindung sieht vor, dass das Messer in eine Messeraufnahme steckbar und dort auswechselbar arretierbar, vorzugsweise mittels einer Rastverbindung einrastbar, ist. Dies ermöglicht ein einfaches Befestigen des Messers sowie ein leichtes Austauschen.

Vorteilhafterweise weist die Rastverbindung eine Bohrung und einen in der Bohrung einrastenden Kugelkopf auf, was eine einfache Arretierung des Messers in der Messeraufnahme ermöglicht.

Vorzugsweise ist die Messeraufnahme um eine Schwenkachse schwenkbar angeordnet und über eine durch das Schaftrohr geführte Betätigungsstange mit dem Abzug verbunden. Dies ermöglicht eine einfache Betätigung des Messers.

Gemäß einer besonders bevorzugten Ausführungsform der Erfindung ist das Messer durch einen Schlitz in einer der Koagulationsplatten in einem Maulteil versenkbar angeordnet. Dadurch ist das Messer in dem Fall, dass gerade nicht geschnitten werden soll, sicher verwahrt, so dass ein unbeabsichtigtes Schneiden vermieden werden kann.

Eine vorteilhafte Ausführungsform der Erfindung sieht vor, dass der Abzug eine Sicherheitseinrichtung aufweist, um ein unbeabsichtigtes Auslösen des Abzugs, welches ein Schneiden des Messers bewirkt, vermeiden zu können.

Vorteilhafterweise weist die Sicherheitseinrichtung einen Rastmechanismus auf, welcher durch eine Bewegung quer zur Bewegung des Abzugs zu überwinden ist, bevor der Abzug betätigt werden kann. Durch die beiden verschiedenen Bewegungsrichtungen zur Bewegung des Abzugs einerseits und zur Lösung der Sicherheitseinrichtung andererseits wird es unwahrscheinlicher, dass der Abzug versehentlich betätigt wird.

Vorteilhafterweise weist der Abzug eine kurvenförmige T-Nut auf, in welcher eine Feder, insbesondere eine kurvenförmige Blattfeder, geführt ist, an welcher ein Rastbolzen angeordnet ist, welcher in einer Position in einer Rastbohrung an der T-Nut einrastet. Vorteilhafterweise erfolgt dabei das Einrasten durch die Feder beaufschlagt, so dass ein Lösen des Rastbolzens aus der Ausnehmung gegen die Kraft der Feder erfolgt und anschließend der Abzug betätigt werden kann derart, dass die Feder, insbesondere die kurvenförmige Blattfeder, in der kurvenförmigen T-Nut verschiebbar geführt wird.

Ein weiteres erfindungsgemäßes bipolares Koagulationsinstrument mit einem Schaftrohr, mit einem am distalen Ende des Schaftrohrs angeordneten Arbeitselement, welches zwei gegeneinander mittels zweier am proximalen Ende des Schaftrohrs angeordneter, gegeneinander verschwenkbarer Griffteile verschwenkbare Maulteile, welche jeweils eine Koagulationsplatte aufweisen, aufweist, zeichnet sich dadurch aus, dass die Koagulationsplatten in den jeweiligen Maulteilen mittels einer Maulteileinlage isoliert angeordnet sind. Dadurch wird es ermöglicht, dass lediglich die isoliert angeordneten Koagulationsplatten mit Strom beaufschlagt werden, so dass eine Isolierung des Schaftrohrs entfallen kann.

Vorteilhafterweise ist die Maulteileinlage schalenförmig ausgebildet, um die Koagulationsplatte abgesehen von der der anderen Koagulationsplatte zugewandten Seitenfläche der Koagulationsplatte vollständig zu umgeben.

Vorzugsweise ist jede der Koagulationsplatten über einen isolierten elektrischen Leiter mit einem am proximalen Ende des Schaftrohrs angeordneten Stromanschluss verbunden, wodurch auf einfache Art und Weise die Koagulationsplatten mit Strom beaufschlagt werden können, ohne dass zusätzliche Maßnahmen zur Isolation an dem Schaftrohr vonnöten sind.

Vorteilhafter Weise ist eine Verriegelungsvorrichtung für das Maulteil vorgesehen, welche die Maulteile in einer Position gegeneinander arretiert, sodass eine Verschwenkung der Maulteile gegeneinander verhindert wird. Dies ermöglicht das Einklemmen von Gewebe zwischen den beiden Maulteilen zur Koagulation und zum anschließenden Schneiden.

Eine bevorzugte Ausführungsform der Erfindung sieht vor, dass die Verriegelungsvorrichtung ein Sperrelement, insbesondere in Form einer Blattfeder, mit einem Rasthaken aufweist, welches an dem Schaftrohr oder einem starr mit dem Schaftrohr verbundenen Element angeordnet ist, wobei der Rasthaken in einen Rastvorsprung eingreift, welcher an dem relativ zum Schaftrohr verschwenkbaren Griffteil oder einem mit dem relativ zum Schaftrohr verschwenkbaren Griffteil verbundenen Element, wie beispielsweise einer Zugstange für das verschwenkbare Maulteil, angeordnet ist. Dies ermöglicht einen besonders kompakten Aufbau der Verriegelungsvorrichtung, welche insbesondere in das Schaftrohr platzsparend integriert werden kann.

Die Erfindung wird anhand der nachfolgenden Figuren ausführlich erläutert. Es zeigen:
- Figur 1: eine perspektivische Ansicht eines Ausführungsbeispiels eines erfindungsgemäßen bipolaren Koagulationsinstruments,
- Figur 2a: den Abzug mit Sicherheitseinrichtung des Koagulationsinstruments gemäß Figur 1,
- Figur 2b: eine Seitenansicht des Abzugs gemäß Figur 2a,
- Figur 2c: einen Schnitt entlang der Linie A-A in Figur 2b,
- Figur 2d: eine Draufsicht auf den Abzug gemäß Figur 2a,
- Figur 2e: einen Schnitt entlang der Linie B-B in Figur 2d,
- Figur 3a: eine teilweise Explosionsdarstellung der Verriegelungsvorrichtung des Koagulationsinstruments gemäß Figur 1,
- Figur 3b: eine Explosionsdarstellung der Verriegelungsvorrichtung gemäß Figur 3a,
- Figur 3c: eine perspektivische Ansicht der Darstellung gemäß Figur 3a,
- Figur 3d: einen schematischen Längsschnitt durch die Verriegelungsvorrichtung gemäß Figur 3a,
- Figur 4a: eine perspektivische Ansicht des Arbeitsendes des Koagulationsinstruments gemäß Figur 1,
- Figur 4b: eine Seitenansicht des Arbeitsendes des Koagulationsinstruments gemäß Figur 1,
- Figur 4c: eine Draufsicht auf das Arbeitsende gemäß Figur 4b,
- Figur 4d: einen Schnitt entlang der Linie A-A in Figur 4c,
- Figur 4e: einen Schnitt entlang der Linie B-B in Figur 4c,
- Figur 4f: einen Schnitt entlang der Linie C-C in Figur 4c und
- Figur 5: eine perspektivische Darstellung der in dem Schaftrohr angeordneten Durchführungsanordnung.

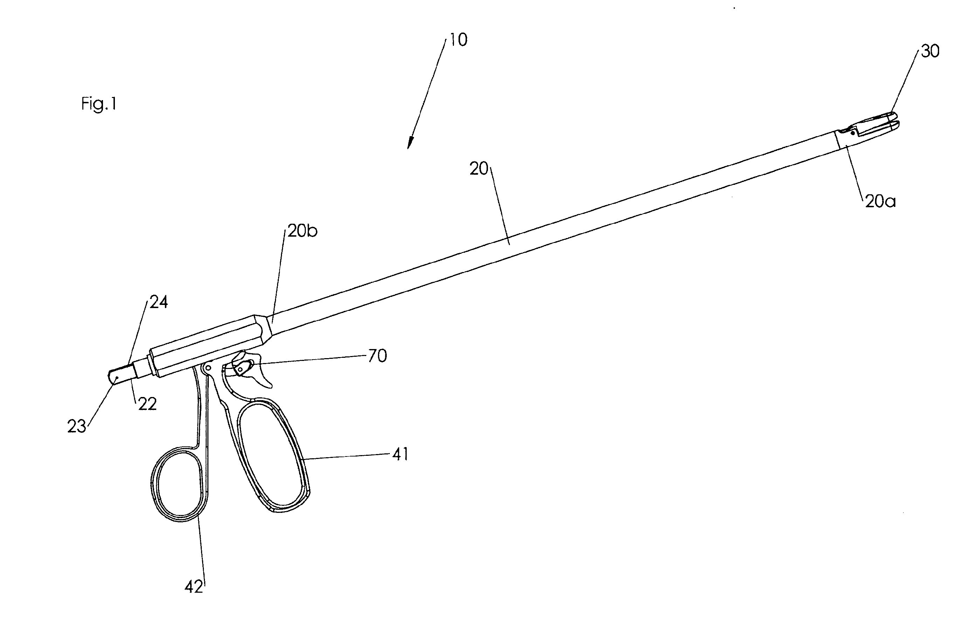

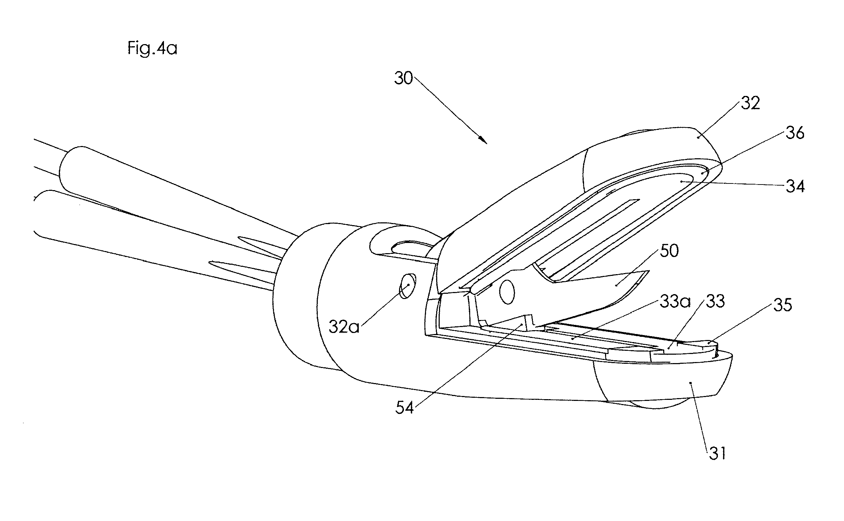

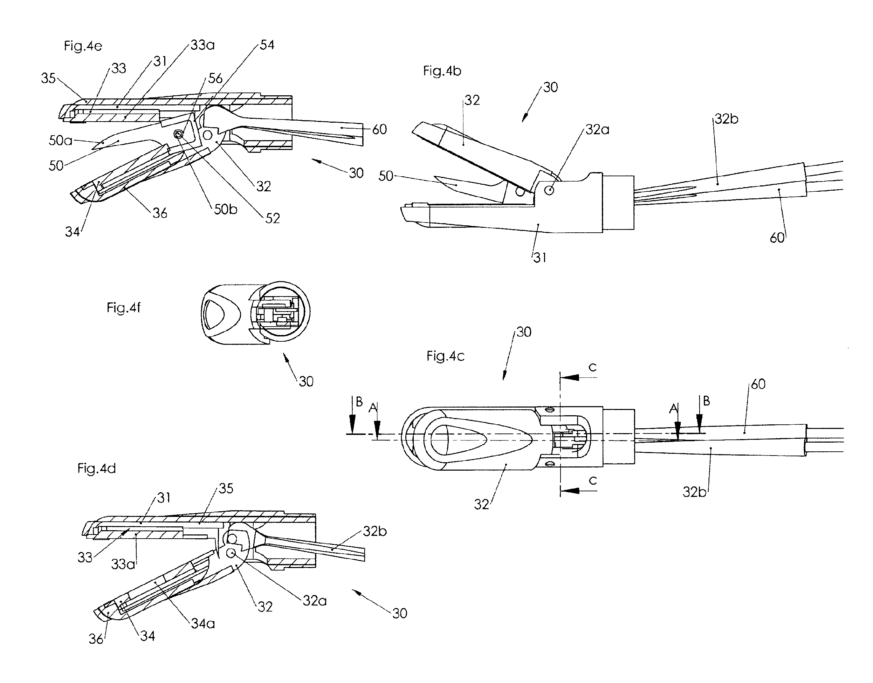

Figur 1 zeigt ein Koagulationsinstrument 10 mit einem Schaftrohr 20, welches ein distales Ende 20a und ein proximales Ende 20b aufweist. Am distalen Ende 20a des Schaftrohrs ist ein Arbeitsende 30 angeordnet, welches ein erstes Maulteil 31 und ein zweites Maulteil 32 aufweist, welche gegeneinander verschwenkbar sind. Das erste Maulteil 31 ist starr mit dem Schaftrohr 20 verbunden. Das zweite Maulteil 32 ist um eine Schwenkachse 32a (vergleiche Figuren 4a bis 4f) schwenkbar gelagert an dem Schaftrohr 20 angeordnet. Das erste Maulteil 31 weist eine erste Koagulationsplatte 33 auf. Das zweite Maulteil 32 weist eine zweite Koagulationsplatte 34 auf. Die erste Koagulationsplatte 33 ist mittels einer Maulteileinlage 35, welche insbesondere schalenförmig ausgebildet sein kann, isolierend in dem ersten Maulteil 31 angeordnet. Die zweite Koagulationsplatte 34 ist mittels einer Maulteileinlage 36, welche ebenfalls schalenförmig ausgebildet sein kann, isoliert in dem zweiten Maulteil 32 angeordnet. Die ersten Koagulationsplatte 33 ist über einen nicht dargestellten elektrischen Leiter mit einem ersten Kontaktelement 23 eines Stromanschlusses 22, welcher am proximalen Ende 20b des Schaftrohrs 20 angeordnet ist, verbunden, während die zweite Koagulationsplatte 34 über einen weiteren nicht dargestellten elektrischen Leiter mit einem zweiten Kontaktelement 24 des Stromanschlusses 22 elektrisch leitend verbunden ist.

Das Koagulationsinstrument 10 weist zwei gegeneinander verschwenkbare Griffteile 41, 42 auf. Dabei kann das erste Griffteil 41 starr mit dem Schaftrohr 20 verbunden sein, während das zweite Griffteil 42 um eine Schwenkachse 44 gegen das erste Griffteil 41 schwenkbar gelagert angeordnet ist. Das zweite Griffteil 42 ist gelenkig mit dem proximalen Ende einer Betätigungsstange 32b verbunden, welche durch das Schaftrohr 20 geführt ist und am distalen Ende die Schwenkachse 32a bildend mit dem zweiten Maulteil 32 verbunden ist. Bei Verschwenken des zweiten Griffteils 42 kann somit eine Zug- oder Schubbewegung auf die Betätigungsstange 32b ausgeübt werden, welche bewirkt, dass das zweite Maulteil 32 um die Schwenkachse 32a verschwenkt wird und dabei die beiden Maulteile 31, 32 derart gegeneinander verschwenkt werden, dass das dadurch gebildete Maul geöffnet oder geschlossen werden kann.

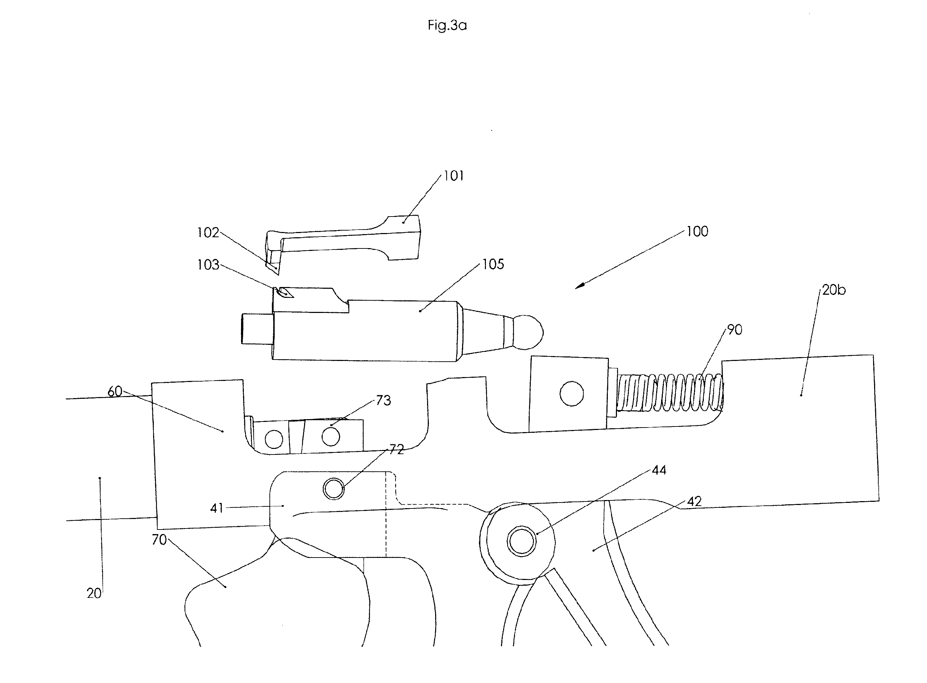

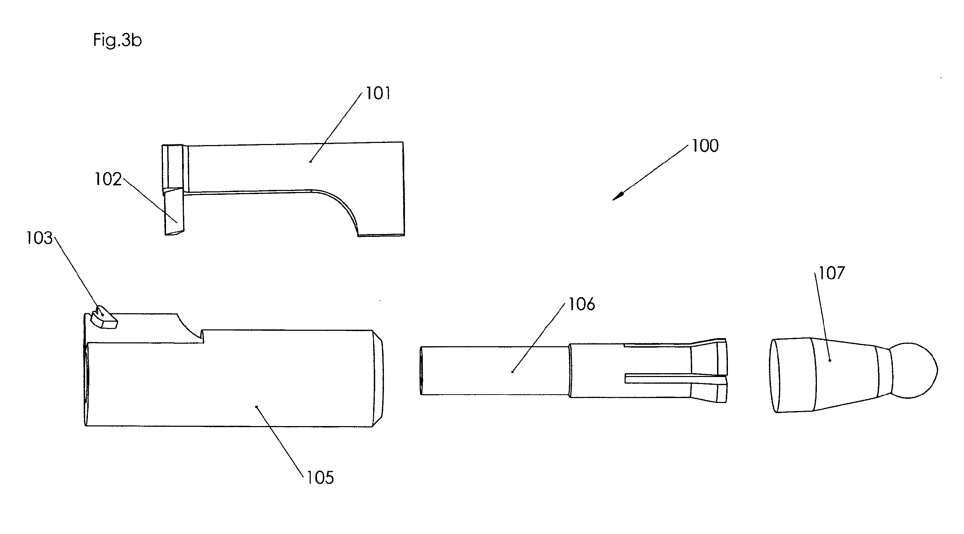

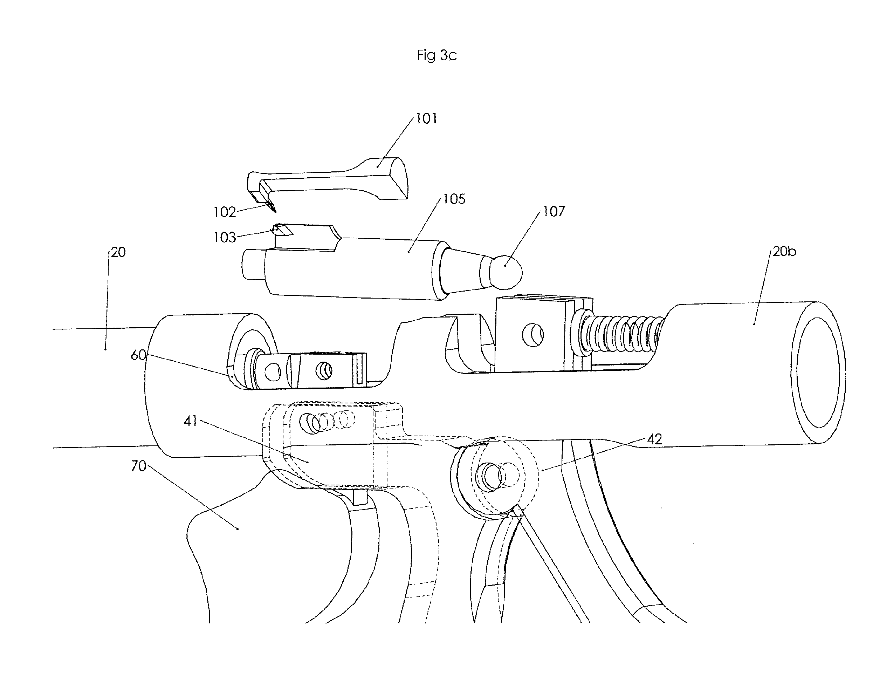

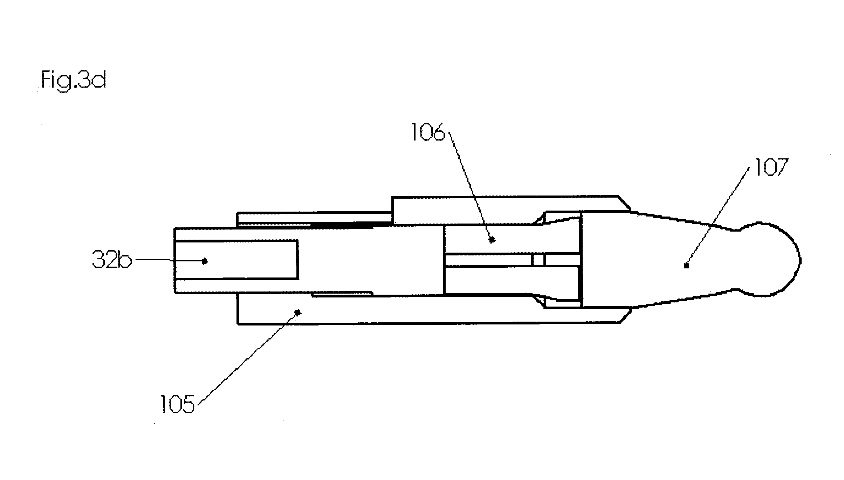

Eine optional vorhandene Verriegelungsvorrichtung 100 für die Schwenkbewegung des zweiten Maulteils 32 ist in den Figuren 3a bis 3d dargestellt. Das proximale Ende der Betätigungsstange 32b wird dabei nicht direkt mit dem schwenkbaren zweiten Griffteil 42 verbunden, sondern die Verbindung erfolgt mittels einer Hülse 105, einer Spannzange 106 und eines Kugelkopfs 107. Das proximale Ende der Betätigungsstange 32b wird in ein distales Ende einer Spannzange 106 eingeführt. Die Spannzange 106 ist in einer Hülse 105 angeordnet. Das proximale Ende der Spannzange 106 wirkt mit einem Verbindungsabschnitt eines Kugelkopfes 107 zusammen. Der Kugelkopf 107 wird in eine entsprechende Ausnehmung an dem schwenkbaren zweiten Griffteil 42 eingelegt. Durch die in der Hülse 105 axial verschiebbare Spannzange 106 ist der Abstand zwischen dem zweiten Griffteil 42 und dem proximalen Ende der Betätigungsstange 32b geringfügig variabel. An der Hülse 105 ist ein Rastvorsprung 103 angeordnet, welcher mit einem Rasthaken 102 eines Sperrelements 101 zusammenwirkt. Das Sperrelement 101 ist vorzugsweise als Blattfeder ausgebildet und an dem Schaftrohr 20 oder einem starr mit dem Schaftrohr 20 verbundenen Element angeordnet.

Wird das zweite Griffteil 42 derart verschwenkt, dass das zweite Maulteil 32 auf das erste Maulteil 31 zugeschwenkt wird, wird das Maul geschlossen und zwischen den beiden Maulteilen 31, 32 liegendes Gewebe in das Maulteil eingespannt. Dabei rastet der Rasthaken 102 an dem Rastvorsprung 103 ein. Bei geschlossenem Maulteil kann koaguliert werden, indem die beiden Koagulationsplatten 33, 34 mit Strom beaufschlagt werden. Nach erfolgter Koagulation kann ein optisches oder akustisches Signal abgegeben werden, um darauf hinzuweisen, dass der Koagulationsvorgang abgeschlossen wurde. Anschließend kann ein Schneidvorgang erfolgen, welcher nachfolgend detaillierter beschrieben wird. Soll das Maulteil wieder geöffnet werden, muss die Rastung zwischen dem Rasthaken 102 und dem Rastvorsprung 103 mit erhöhtem Druck über die Spannzange 106 zum Umlauf gebracht werden und insbesondere der Rasthaken 102 außer Eingriff mit dem Rastvorsprung 103 gebracht werden. Durch den erhöhten Druck auf die Spannzange 106 gibt die Spannzange 106 in axialer Richtung so lange nach, bis die Rastverbindung zwischen dem Rasthaken 102 und dem Rastvorsprung 103 gelöst wird. Das Maul kann nun wieder geöffnet werden.

In einer Ausführungsform ist das zweite Griffteil 42 derart mit einem Federelement 90 beaufschlagt, welches beispielsweise zwischen dem zweiten Griffteil 42 und dem distalen Ende 20a des Schaftrohrs 20 angeordnet ist, dass das Federelement 90, sofern eine Öffnung des Mauls nicht durch die Verriegelungsvorrichtung 100 verhindert ist, durch das Federelement 90 beaufschlagt in vom Benutzer unbelastetem Zustand automatisiert erfolgt.

Das Koagulationsinstrument 10 kann ein Messer 50 mit einem distalen Ende 50a und einem proximalen Ende 50b aufweisen, welches in dem Arbeitsende 30 angeordnet ist. Das Messer 50 kann um eine Schwenkachse 58 schwenkbar gelagert in dem Arbeitsende 30 angeordnet sein. Damit das Messer 50 bei einem Koagulationsvorgang nicht stört, kann das Messer 50 durch einen Schlitz 33a in der ersten Koagulationsplatte 33 in das erste Maulteil 31 derart versenkt werden, dass es nicht mehr in den Zwischenraum zwischen der ersten Koagulationsplatte 33 und der zweiten Koagulationsplatte 34 hineinragt. Alternativ kann das Messer 50 auch in dem beweglichen zweiten Maulteil 32 versenkbar sein. Bei einem Schneidvorgang wird das Messer 50 um die Schwenkachse 58 geschwenkt. Damit insbesondere ein Durchschneiden auch bei geschlossenem Maulteil möglich ist, weist die zweite Koagulationsplatte 34 einen Schlitz 34a auf, in den das Messer 50 beim Durchschneiden eindringen kann.

Das Messer 50 ist auswechselbar in dem Arbeitsende 30 angeordnet, insbesondere derart, dass es durch das Maulteil vom distalen Ende des Koagulationsinstruments 10 her entnommen werden kann, insbesondere ohne dass das Koagulationsinstrument 10 vollständig demontiert werden muss oder das Messer 50 durch das Schaftrohr 20 zum proximalen Ende hin entnommen werden muss. Dazu ist das Messer mit seinem proximalen Ende 50b in einer Messeraufnahme 54 angeordnet, welche um die Schwenkachse 58 schwenkbar angeordnet ist. Das Messer 50 ist in die Messeraufnahme 54 einsteckbar und dort arretierbar, insbesondere verrastbar. Dazu kann die Messeraufnahme 54 einen Kugelkopf 52 aufweisen, welcher in eine Bohrung 56 des Messers 50 rastend eingreift. Die Messeraufnahme 54 ist um die Schwenkachse 58 schwenkbar mit einer Betätigungsstange 60 verbunden, welche durch das Schaftrohr 20 geführt ist und am proximalen Ende 20b des Schaftrohrs 20 mittels eines Abzugs 70 betätigbar ist. Der Abzug 70 ist dazu insbesondere über eine Schwenkachse 72 schwenkbar gelagert an dem Schaftrohr 20 oder, wie in der Figur 3a dargestellt, an dem feststehenden ersten Griffteil 41 angeordnet. Die schwenkbare Verbindung zwischen dem Abzug 70 und der Betätigungsstange 60 erfolgt über die schwenkbare Verbindung 73. Wird der Abzug 70 um die Schwenkachse 72 verschwenkt, wird die Betätigungsstange 60 in axialer Richtung in dem Schaftrohr 20 vor- oder zurückbewegt und darüber das Messer 50 um die Schwenkachse 58 verschwenkt, um einen Schneidvorgang auszulösen.

Damit der Abzug 70 nicht versehentlich verschwenkt wird, kann eine Sicherheitseinrichtung vorgesehen werden, welche die Bewegung des Abzugs 70 um die Schwenkachse 72 blockiert. Dazu kann der Abzug 70 eine T-förmige Nut 74 aufweisen, welche insbesondere gekrümmt ausgebildet ist. In der Nut 74 ist eine Feder 80 geführt verschiebbar angeordnet, welche insbesondere als Blattfeder ausgebildet ist. Die Feder 80 weist ein erstes Ende 80a und ein zweites Ende 80b auf, wobei das zweite Ende 80b an einem feststehenden Element des Koagulationsinstruments 10, beispielsweise dem ersten Griffteil 41, fixiert ist. Das zweite Ende 80b ist bei Bewegung des Abzugs 70 in der Nut 74 verschiebbar. An dem zweiten Ende 80b der Feder 80 ist ein Rastbolzen 82 angeordnet, welcher durch die Nut 74 auf die Außenseite des Abzugs 70 geführt ist. An dem freien Ende des Rastbolzens 82 kann ein Druckknopf 84 angeordnet sein.

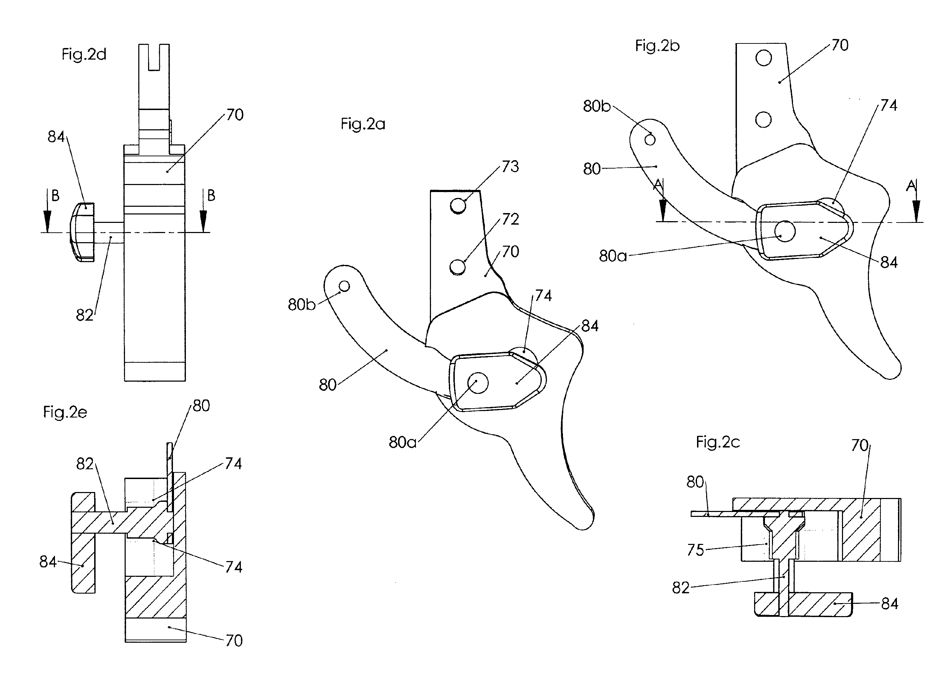

Die T-förmige Nut 74 weist eine Rastbohrung 75 auf, in welcher der Rastbolzen 82 in der verriegelten Position einrastet. Ein Verschieben des Rastbolzens 82 entlang der Nut 74 ist nicht möglich, sodass die Bewegung des Abzugs 70 um die Schwenkachse 72 blockiert ist. Wird der Rastbolzen 82 gegen die Federkraft der blattfederartigen Feder 80 in die Nut 74 eingedrückt, kommt der Rastbolzen 82 außer Eingriff mit der Rastbohrung 75 (vergleiche Figuren 2c und 2e), sodass ein Verschieben des Rastbolzens 82 entlang der Nut 74 möglich ist. Somit kann der Abzug 70 um die Schwenkachse 72 verschwenkt werden, um einen Schneidvorgang mit dem Messer 50 auszulösen.

Vorteilhafterweise ist auch der Abzug 70 derart federbelastet, dass er im nicht durch einen Benutzer belasteten Zustand durch die Kraft der Feder in die Ausgangsposition überführt wird, in welcher das Messer 50 in dem ersten Maulteil 31 versenkt angeordnet ist und die Sicherheitseinrichtung in Eingriff ist.

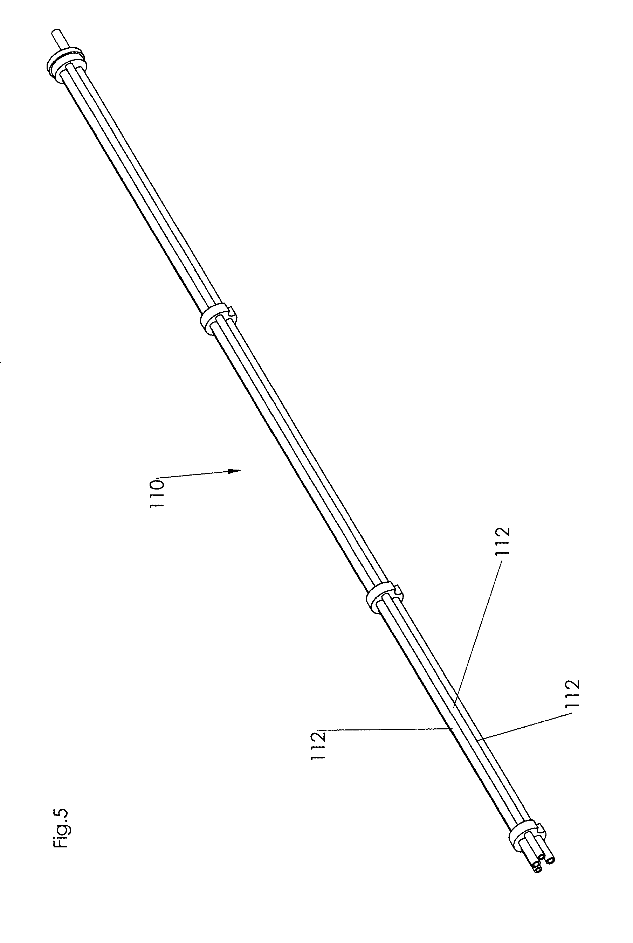

Durch das Schaftrohr 20 sind zwei elektrische Leiter sowie die Betätigungsstange 60 zur Bewegung des Messers 50 und die Betätigungsstange 32b zur Bewegung des zweiten Maulteils 32 geführt. Um einen Kontakt zwischen den elektrischen Leitern und den Betätigungsstangen 60, 32b zu vermeiden und vorzugsweise eine Abschirmung der elektrischen Leiter gegeneinander zu erreichen, kann in einer Ausführungsform in dem Schaftrohr 20 eine Rohranordnung 110 mit beispielsweise vier parallel zueinander angeordneten Rohren 112 angeordnet sein (vergleiche Figur 5), wobei in jedem der Rohre 112 genau eines der Elemente der zwei elektrischen Leiter, der Betätigungsstange 32b für das zweite Maulteil 32 und der Betätigungsstange 60 für das Messer 50 angeordnet ist.

### Bezugszeichenliste

- 10: Koagulationsinstrument
- 20: Schaftrohr
- 20a: distales Ende
- 20b: proximales Ende
- 22: Stromanschluss
- 23: Kontaktelement
- 24: Kontaktelement
- 30: Arbeitsende
- 31: erstes Maulteil
- 32: zweites Maulteil
- 32a: Schwenkachse
- 32b: Betätigungsstange
- 33: erste Koagulationsplatte
- 33a: Schlitz
- 34: zweite Koagulationsplatte
- 34a: Schlitz
- 35: erste Maulteileinlage
- 36: zweite Maulteileinlage
- 41: erstes Griffteil
- 42: zweites Griffteil
- 44: Schwenkachse
- 50: Messer
- 52: Kugelkopf
- 54: Messeraufnahme
- 56: Bohrung
- 58: Schwenkachse
- 60: Betätigungsstange
- 70: Abzug
- 72: Schwenkachse
- 73: Verbindung
- 74: Nut
- 80: Feder
- 80a: erstes Ende
- 80b: zweites Ende
- 82: Rastbolzen
- 84: Druckkopf
- 90: Federelement
- 100: Verriegelung
- 101: Sperrelement
- 102: Rasthaken
- 103: Rastvorsprung
- 105: Hülse
- 106: Spannzange
- 107: Kugelkopf
- 110: Rohranordnung
- 112: Rohr

## Patentansprüche

1. Bipolares Koagulationsinstrument (10) mit einem Schaftrohr (20), mit einem am distalen Ende (20a) des Schaftrohrs (20) angeordneten Arbeitselement 830), welches zwei gegeneinander mittels zweier am proximalen Ende (20b) des Schaftrohrs (20) angeordneter, gegeneinander verschwenkbarer Griffteile (41, 42) verschwenkbare Maulteile (31, 32), welche jeweils eine Koagulationsplatte (33, 34) aufweisen, und ein mittels eines am proximalen Ende (20b) des Schaftrohrs (20)) angeordneten Abzugs (70) bewegbares Messer (50) aufweist,
**dadurch gekennzeichnet, dass** das Messer (50) auswechselbar ist.

2. Bipolares Koagulationsinstrument nach Anspruch 1,
**dadurch gekennzeichnet, dass** das Messer (50) vom distalen Ende her auswechselbar ist.

3. Bipolares Koagulationsinstrument nach einem der vorhergehenden Ansprüche,
**dadurch gekennzeichnet, dass** das Messer (50) in eine Messeraufnahme (54) steckbar und dort auswechselbar arretierbar, vorzugsweise mittels einer Rastverbindung einrastbar, ist.

4. Bipolares Koagulationsinstrument nach Anspruch 3,
**dadurch gekennzeichnet, dass** die Rastverbindung eine Bohrung (56) und einen in der Bohrung (56) einrastenden Kugelkopf (52) aufweist.

5. Bipolares Koagulationsinstrument nach Anspruch 3 oder 4,
**dadurch gekennzeichnet, dass** die Messeraufnahme (54) um eine Schwenkachse (58) schwenkbar angeordnet ist und über eine durch das Schaftrohr (20) geführte Betätigungsstange (60) mit dem Abzug (70) verbunden ist.

6. Bipolares Koagulationsinstrument nach einem der vorhergehenden Ansprüche,
**dadurch gekennzeichnet, dass** das Messer (50) um eine Schwenkachse (58) verschwenkbar angeordnet ist.

7. Bipolares Koagulationsinstrument nach einem der vorhergehenden Ansprüche,
**dadurch gekennzeichnet, dass** das Messer (50) durch einen Schlitz (33a) in einer der Koagulationsplatten (33) in einem der Maulteile (31) versenkbar angeordnet ist.

8. Bipolares Koagulationsinstrument nach einem der vorhergehenden Ansprüche,
**dadurch gekennzeichnet, dass** der Abzug (70) eine Sicherheitseinrichtung aufweist.

9. Bipolares Koagulationsinstrument nach Anspruch 8,
**dadurch gekennzeichnet, dass** die Sicherheitseinrichtung einen Rastmechanismus aufweist, welcher durch eine Bewegung quer zur Bewegung des Abzugs (70) zu überwinden ist, bevor der Abzug (70) betätigbar ist.

10. Bipolares Koagulationsinstrument nach Anspruch 8 oder 9,
**dadurch gekennzeichnet, dass** der Abzug (70) eine kurvenförmige T-Nut (74)aufweist, in welcher eine Feder (80), insbesondere eine kurvenförmige Blattfeder geführt ist, an welcher ein Rastbolzen (82) angeordnet ist, welcher in einer Position in eine Rastbohrung (75) an der T-Nut (74) einrastet.

11. Bipolares Koagulationsinstrument, insbesondere nach einem
der vorhergehenden Ansprüche, mit einem Schaftrohr (20), mit einem am distalen Ende (20a) des Schaftrohrs (20) angeordneten Arbeitselement (30), welches zwei gegeneinander mittels zweier am proximalen Ende (20b) des Schaftrohrs (20) angeordneter, gegeneinander verschwenkbarer Griffteile (41, 42) verschwenkbare Maulteile (31, 32), welche jeweils eine Koagulationsplatte (33, 34) aufweisen,
**dadurch gekennzeichnet, dass** die Koagulationsplatten (33, 34) in den jeweiligen Maulteilen (31, 32) mittels einer Maulteileinlage (35, 36) isoliert angeordnet sind.

12. Bipolares Koagulationsinstrument nach Anspruch 11,
**dadurch gekennzeichnet, dass** die Maulteileinlage (35, 36) schalenförmig ausgebildet ist.

13. Bipolares Koagulationsinstrument nach Anspruch 11 oder 12,
**dadurch gekennzeichnet, dass** jede der Koagulationsplatten (33, 34) über einen isolierten elektrischen Leiter mit einem am proximalen Ende (20b) des Schaftrohrs (20) angeordneten Stromanschluss (22) verbunden ist.

14. Bipolares Koagulationsinstrument nach einem der vorhergehenden Ansprüche,
**dadurch gekennzeichnet, dass** eine Verriegelungsvorrichtung (100) für das Maulteil vorgesehen ist.

15. Bipolares Koagulationsinstrument nach einem der vorhergehenden Ansprüche,
**dadurch gekennzeichnet, dass** die Verriegelungsvorrichtung (100) ein Sperrelement (101), insbesondere in Form einer Blattfeder, mit einem Rasthaken (102) aufweist, welches an dem Schaftrohr (20) oder einem starr mit dem Schaftrohr (20) verbundenen Element angeordnet ist, wobei der Rasthaken (102) an einem Rastvorsprung (103) angreift, welcher an dem relativ zum Schaftrohr (20) verschwenkbaren Griffteil (42) oder einem mit dem relativ zum Schaftrohr (20) verschwenkbaren Griffteil (42) verbundenen Element angeordnet ist.
